(19) Europäisches Patentamt
European Patent Office
Office européen des brevets

(11) Numéro de publication: **0 187 052 B1**

# (12) FASCICULE DE BREVET EUROPEEN

(45) Date de publication du fascicule du brevet: **31.01.90**

(51) Int. Cl.⁴: **C 07 D 207/28**, A 61 K 31/40

(21) Numéro de dépôt: **85401976.7**

(22) Date de dépôt: **10.10.85**

(54) **Nouveaux dérivés de l'adamantanamine, leurs procédés de préparation et médicaments les contenant.**

(30) Priorité: **31.10.84 FR 8416656**

(43) Date de publication de la demande: **09.07.86 Bulletin 86/28**

(45) Mention de la délivrance du brevet: **31.01.90 Bulletin 90/5**

(84) Etats contractants désignés: **AT BE CH DE IT LI LU NL SE**

(56) Documents cités:
**EP-A- 0 098 520**
**FR-M- 2 622**
**US-A- 3 152 180**
**US-A- 4 273 704**

**DIE PHARMAZIE, vol. 30, no. 9, septembre 1975, pages 571-581, Berlin, DE; F. SZTARICSKAI et al.: "Synthese und virushemmende in-vitro-Wirkung neuerer 1-substituierter Adamantanderivate"**
**CHEMICAL ABSTRACTS, vol. 102, no. 9, 4 mars 1985, page 39, no. 72572k, Columbus, Ohio, US; K. MASEK et al.: "The immunomodulatory property of a novel synthetic compound adamantylamide dipeptide", & METHODS FIND. EXP. CLIN. PHARMACOL. 1984, 6(11), 667-9**
**CHEMICAL ABSTRACTS, vol. 95, no. 3, 20 juillet 1981,**

(73) Titulaire: **S.A. PANMEDICA Société dite:, Zone Industrielle - Ilot J, F-06510 Carros (FR)**

(72) Inventeur: **Laruelle, Claude, Avenue Bellevue, F-06270 Villeneuve Loubet (FR)**
Inventeur: **Lepant, Marcel, 7 rue Mellarède, F-06100 Nice (FR)**
Inventeur: **Raynier, Bernard, 9 Chemin du Malvan "Les Glycines", F-06800 Cagnes S/Mer (FR)**

(74) Mandataire: **Orès, Bernard et al, Cabinet ORES 6, Avenue de Messine, F-75008 Paris (FR)**

(56) Documents cités: (suite)
**pages 661-662, no. 24693r, Columbus, Ohio, US; O.M. GLOZMAN et al.: "Synthesis and anticonvulsive activity of 4-phenyl-2-pyrrolidinone-1-acetic acid amides", & KHIM.-FARM. ZH. 1980, 14(11), 43-48**
**CHEMICAL ABSTRACTS, vol. 98, no. 15, 11 avril 1983, page 613, no. 125804v, Columbus, Ohio, US; N.G. ARTSIMOVICH: "N-Adamantyl derivatives of pyrrolidone and their biological activity", & KHIM.-FARM. ZH. 1982, 16(10), 1197-201**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

EP 0 187 052 B1

**Description**

La présente invention est relative à de nouveaux dérivés de l'adamantanamine, à leur procédé de préparation et aux médicaments les contenant.

Parmi les composés hydrocarbonés pontés présentant une activité pharmacologique (D.L. Swallow, Progr. Med. Chem. [1971] 119), l'adamantanamine a été plus particulièrement étudiée pour son action prophylactique et thérapeutique sur virus Influenza (Brevet Américain 3 152 180). Des effets centraux ont été d'autre part mis en évidence par R.S. Swab, A.C. England, D.C. Poskanzer, R.R. Yong (J. Am. Med. Assoc. 208 [1969] 1168), dans le traitement de la maladie de Parkinson.

Plus récemment, la demande européenne EP 98 520 revendique les activités immunostimulantes des L-Alanyl-D-isoglutaminyl-adamantylamides.

L'utilisation prophylactique et thérapeutique de l'adamantanamine est connue depuis longtemps mais sa durée d'action et l'apparition d'effets secondaires tels que, par exemple, insomnies, œdèmes des membres inférieurs, troubles neuropsychiques, circulatoires ou trophiques limitent malheureusement son usage à des personnes à haut risque vis-à-vis de virus Influenza.

La présente invention s'est, par conséquent, fixée pour but de pourvoir à des nouveaux dérivés de l'adamantanamine ne présentant pas ou peu d'effets secondaires tout en augmentant la durée d'action immunostimulante de l'adamantanamine.

La présente invention a pour objet de nouveaux dérivés de l'adamantanamine, répondant à la formule générale I ci-après:

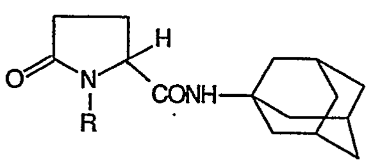 (I)

dans laquelle:

- R représente l'hydrogène, ou un radical alkyle linéaire ou ramifié comprenant de 1 à 7 atomes de carbone, substitué ou non par un groupement alkoxyle, thioalkyle ou nitrile, ou des radicaux acyle du type

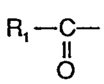 ou 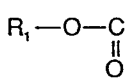

où

- $R_1$ représente un radical alkyle linéaire ou ramifié, comprenant de 1 à 7 atomes de carbone, un radical cycloalkyle comprenant 5 à 7 atomes de carbone, le cycle en question pouvant être relié au radical carbonyle par un ou deux groupements méthylène, un noyau aromatique, éventuellement substitué par des substituants tels que l'atome d'halogène, les groupes nitro, alkoxyle ou alkyle de $C_1$ à $C_3$, un radical benzylique éventuellement substitué par des substituants tels que l'atome d'halogène, le groupe nitro, alkoxyle ou alkyle de $C_1$ à $C_3$.

La présente invention a également pour objet la préparation des composés répondant à la formule générale I. La préparation peut faire intervenir la réaction de l'acide (L) pyroglutamique N-substitué correspondant avec l'adamantanamine, en présence d'un agent de couplage par exemple le N,N'dicyclohexylcarbodiimide ou le N éthyl, N' (diméthylaminopropyl) carbodiimide, dans un solvant inerte. La préparation peut également impliquer un dérivé réactif de l'acide (L) pyroglutamique N-substitué. Parmi les dérivés réactifs susceptibles d'être employés, on peut retenir les halogénures d'acide, les esters paranitrophényle, pentachlorophényle, les anhydrides mixtes, préparés selon des réactions connues, utilisant des esters chloroformiques, le chloroformiate d'éthyle plus généralement, ou des chlorures d'acides tels le chlorure de pivaloyle.

On pourra employer comme solvants des haloalkanes, des éthers, des amines tertiaires, des amides, seuls ou mélangés, et plus précisément la pyridine, le tetrahydrofuranne, l'acétonitrile, le N,N diméthylformamide, le N,N diméthylacétamide, le chlorure de méthylène, le chloroforme.

Dans le cas où le dérivé réactif est un halogénure d'acide, on utilisera avantageusement un accepteur d'acide, plus fréquemment la pyridine ou la triéthylamine.

Lorsque R représente l'hydrogène, la N-substitution de l'acide (L) pyroglutamique est réalisée à partir de l'acide (L) glutamique, par l'intermédiaire de l'acide (L) glutamique N-substitué, de l'anhydride (L) glutamique N-substitué, et du sel de dicyclohexylamine de l'acide (L) pyroglutamique N-substitué, selon le procédé décrit par H. Gibian et D. Klieger (Ann. 640, [1961], 145-156).

Après obtention des produits de formule générale I

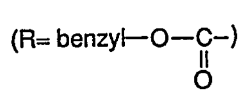

on peut éliminer, si on le désire, le groupe N-protecteur par hydrogénolyse, généralement dans un solvant hydroalcoolique, en présence de palladium sur charbon, pour obtenir le produit I (R = H).

Lorsque R représente un radical alkyle ou

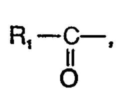

$R_1$ ayant la même signification que précédemment, la N-substitution de l'acide (L) pyroglutamique est réalisée par alkylation ou acylation du sel sodique de l'ester benzylique de l'acide (L) pyroglutamique, suivie d'une hydrogénation catalytique au moyen de charbon palladié. Le sel sodique est généralement préparé in situ par l'intermédiaire d'hydrure ou de méthylate de sodium, dans un solvant inerte pouvant être du benzène, du tetrahydrofuranne, du N,N diméthyl-formamide. L'hydrogénolyse de l'ester benzylique est effectuée de préférence dans un solvant hydroalcoolique, plus spécialement dans l'éthanol.

Les exemples qui suivent sont des illustrations des composés de formule générale I et des diverses voies d'accès détaillées précédemment, mais ne limitent en rien la portée de la présente invention. Tous les produits obtenus ont été soumis à l'analyse par chromatographie en couche mince et ne présentent qu'un seul spot. Les chromatographies sur couche mince (CCM) ont été réalisées sur plaque Kieselgel F 254 et développées, pour les produits de formule générale I, dans les systèmes suivants:

A: toluène 10; acide formique 1; formiate d'éthyle 10

B: $CHCl_3$ 95 – acétone 5

et révélées en lumière ultra-violette à 254 nm. Les résultats des analyses centésimales pratiquées sur tous les produits sont conformes aux formules théoriques.

Tous les produits obtenus outre une activité antivirale très prononcée, présentent également des propriétés hypocholestérolémiantes, hypobétalipoprotéinémiantes et anticonvulsivantes.

Exemples de préparation

Exemple I

N-[Adamantyl-1] (L) Pyroglutamide (I; R = H)

1) Par l'intermédiaire d'un chlorure d'acide pyroglutamique $N^1$-protégé

a) Acide $N^1$-benzyloxycarbonyl (L) glutamique

On dissout 400 g (2,72 M) d'acide (L) glutamique dans 2,66 l de soude 2 N, on refroidit à 5 °C et on coule lentement environ 400 ml de chloroformiate de benzyle (3,6 Moles) en ajoutant simultanément de la soude diluée (1 à 2 N) pour maintenir le pH vers 9,0. On laisse agiter quelques heures, lave avec 2 × 200 ml d'éther et acidifie vers pH 1. Après extraction à l'acétate d'éthyle, lavages à l'eau saturée en chlorure de sodium, séchage et évaporation, on obtient une huile épaisse qui cristallise dans l'hexane.

Poids 500 g; Rdt = 65% – pF 116/7 °C

Titre $H^+$ = 99%: $[\alpha]_D^{22} = -10{,}4°$ (1%, acétone).

b) Anhydride N1-benzyloxycarbonyl-(L) glutamique.

On dissout à température ambiante 500 g d'acide NCbo-glutamique (1,78 Mole) dans 3,5 l de THF, ajoute sous agitation 361 g de dicyclohexylcarbodiimide, dissout dans 1,75 l de THF. La dicyclohexylurée précipite rapidement, on laisse agiter une nuit puis filtre l'urée. On évapore à sec le filtrat et on reprend l'huile épaisse résiduelle par 6 à 8 fois son poids d'un mélange Ether 90 Chloroforme 10. Après quelques jours au réfrigérateur, on filtre les cristaux formés. Poids sec ⩾ 400 g – Rdt = 85% à 90% – pF 90/91 °C $[\alpha]_D^{22} = -41{,}5°$ (2%, acide acétique).

c) Sel de dicyclohexylamine de l'acide N1-benzyloxycarbonyl (L)-pyroglutamique.

On met en suspension à température ordinaire 400 g (1,52 Mole) d'anhydride $N^1$-benzyloxycarbonyl (L) glutamique dans 1,5 l de THF et 4 l d'éther et coule très lentement une solution de 300 ml de dicyclohexylamine (1,52 Mole) dans 500 ml d'éther.

Le sel de Dicyclohexylamine (DCHA) précipite au fur et à mesure de la coulée. Après une nuit d'agitation, on filtre et on sèche.

Le rendement est quantitatif en produit blanc de pF 206/208 °C. On recristallise les 735 g de produit brut dans 7 à 8 volumes d'éthanol.

d) Chlorure de l'acide $N^1$-benzyloxycarbonyl (L)-pyroglutamique.

On empate 50 g (0,113 Mole) de sel de dicyclohexylamine de l'acide $N^1$-benzyloxycarbonyl (L) pyroglutamique précédemment obtenu dans 1 litre de toluène et coule goutte à goutte 110 ml de chlorure de thionyle et on porte ensuite la température à 60 °C pendant 2 heures. On refroidit, filtre et le filtrat évaporé est repris plusieurs fois au benzène. Le chlorure d'acide cristallise finalement dans l'éther de pétrole avec un rendement pratiquement quantitatif. pF 65 à 70 °C (littérature = 71/72 °C).

e) N1-benzyloxycarbonyl N-(adamantyl-1) (L)-pyroglutamide.

On dissout à température ordinaire 25 g (0,165 Mole) d'adamantanamine et 17 ml de triéthylamine dans 450 ml de chloroforme et coule lentement en 3 heures, 45 g (0,16 Mole) du chlorure de l'acide $N^1$-(Cbo) pyroglutamique précédemment préparé en solution dans 450 ml de chloroforme. On contrôle la fin de réaction par CCM, on lave la couche organique à l'eau, à la soude diluée, à l'acide chlorhydrique dilué puis à nouveau à l'eau. Après séchage, on évapore le chloroforme et recristallise le brut dans 600 ml d'éthanol. On obtient ainsi 55% de produit de pF 186/7 °C et 13% de produit de pF 182/3 °C.

f) N-(adamantyl-1) (L)-pyroglutamide.

On hydrogène à pression et température ordinaires 35 g de $N^1$-benzyloxycarbonyl N-(adamantyl-1) pyroglutamide dans 350 ml d'éthanol en présence de 2 g de palladium/charbon. A la fin de l'hydrogénation, on filtre, évapore et recristallise dans un mélange acétate d'éthyle/acétone, on obtient ainsi 78% de produit pur de pF = 194/5 °C $[\alpha]_D^{22} = -56°$ (C = 1, chloroforme) et présentant les caractéristiques suivantes: I.R.: NH 3310 cm−1 FF; CONH cm−1 FF; 1640 cm−1 et 1530 cm−1.

R.M.N.: NH (2 H) (s) à 7,6 et 6,1 ppm; CH—CO (m) 1 H à 4,2 ppm; $CH_2$—$CH_2$ (pyrrolidone) (m) (4 H) à 2,4 ppm – adamantane (2m) 15 H à 2,2 et 1,8 ppm.

2) Par l'intermédiaire des esters réactifs de l'acide (L) pyroglutamique $N^1$-substitués.

a) Ester paranitrophénylique de l'acide $N^1$-(benzyloxycarbonyl) (L) pyroglutamique.

On dissout 10 g (40 mM) d'acide $N^1$-benzyloxycarbonyl) (L) pyroglutamique, 5,84 g (40 mM) de p-nitrophénol, dans 100 ml de THF. On coule à température ordinaire une solution de 8,65 g (42 mM) de N,N dicyclohexylcarbodiimide dans 40 ml de THF, agite 6 heures, filtre, évapore, reprend à

l'acétate d'éthyle, filtre, évapore et recristallise dans le même solvant. On obtient ainsi 60% d'un produit pur, pF = 141/142°C-$[\alpha]_D^{25}$ = −48,7° (C = 1, THF).

b) N¹-benzyloxycarbonyl N-(adamantyl-1) (L)-pyroglutamide.

On agite pendant 8 heures, 20 mM de l'ester réactif précédent et 21 mM d'adamantanamine, dans un mélange de 20 ml de N,N diméthylformamide et 80 ml de tetrahydrofuranne. On filtre, évapore à sec et recristallise dans l'éthanol comme précisé dans l'exemple 1e). On achève la préparation selon 1f), pour obtenir le produit recherché, avec un rendement voisin de 60%.

On peut de la même façon, obtenir l'ester pentachlorophénylique correspondant et l'utiliser pour obtenir l'amide décrit en 2b), puis l'amide décrit en 1f) avec un rendement voisin de 55%.

3) Par l'intermédiaire d'un anhydride mixte de l'acide (L) pyroglutamique non protégé (R = H).

a) Utilisation du chlorure de pivaloyle: $(CH_3)_3C-COCl$.

On mélange 10 g d'acide (L) pyroglutamique (77 mM), 10,7 ml de triéthylamine, dans 100 ml d'acétone anhydre, et à −5°, on coule lentement 9,29 g de chlorure de pivaloyle (77 mM), puis après 15 mn à 0°C, on ajoute en plusieurs portions 11,65 g (77 mM) d'adamantanamine.

Le milieu très épais, se fluidifie lentement, et après 5 heures à 0°C, on évapore le solvant, reprend au chloroforme et lave abondamment à l'eau. Après séchage et évaporation, le résidu est recristallisé dans l'acétone. On obtient avec 30% de rendement un produit dont les caractéristiques physiques et spectroscopiques correspondent à celles notées dans l'exemple 1f).

Exemple II

N1-Acetyl N-(Adamantyl-1) (L) Pyroglutamide

1) (L) pyroglutamate de benzyle.

Dans un appareil de 4 litres, équipé d'une bonne agitation et d'un Deanstark, on porte au reflux 103,2 g (0,8 Mole) d'acide (L) pyroglutamique, en suspension dans 2,8 litres de toluène, 86,6 g (0,8 Mole) d'alcool benzylique et 4,3 ml d'acide sulfurique, pendant 7 à 8 heures. On vérifie par CCM (A) que l'alcool benzylique a disparu, on refroidit, on lave par 2×400 ml d'eau, 2×400 ml d'une solution de $Na_2CO_3$ 2%, puis par 2×400 ml d'eau. La couche toluénique est séchée sur sulfate de sodium, évaporée sous vide, séchée sous vide en présence de $P_2O_5$. On obtient 70 à 75% d'un produit pur, Rf = 0,5 (A), pF = 48/49°C. On peut également distiller le produit, point d'ébullition = 170–175°C (0,1 mmHg).

2) N1-acétyl (L) pyroglutamate de benzyle.

A une suspension de 13,4 g de NaH (280 mM) dans 50 ml de benzène sec, on ajoute en 2 heures, une solution du (L) pyroglutamate de benzyle, 61,3 g (280 mM), dans 400 ml de benzène. Après agitation, pendant une heure à température ordinaire, on coule une solution de chlorure d'acétyle, 22,7 g (290 mM), dans 150 ml de benzène, on chauffe à 55° pendant 5 heures, puis agite 24 heures à température ordinaire, lave à l'eau, 2×150 ml, sèche et évapore. Le produit est distillé sous pression réduite, point d'ébullition = 156–160°C (0,25 mmHg). On obtient 60% d'un produit pur, Rf = 0,63 (A).

3) Acide N¹-acétyl (L) pyroglutamique.

On hydrogène à pression atmosphérique le produit précédemment obtenu, 43,6 g (167 mM) dans 400 ml d'éthanol, en présence de 2 g de palladium à 5%/C. Après filtration du catalyseur, on évapore à sec, reprend au benzène, évapore à sec, et sèche sous vide poussé pendant 24 heures. On obtient, avec un rendement quantitatif, un produit pur huileux, Rf = 0,33 (A), révélation UV 254 nm et iode.

Ce produit peut être transformé en sel de dicyclohexylamine, recristallisable dans un mélange éthanolacétate d'éthyle, pF = 172/173°C.

4) N¹-acétyl N-(adamantyl-1) (L) pyroglutamide.

L'acide précédemment obtenu, 15,4 g (90 mM), en solution dans 40 ml de chloroforme pur, est coulé sur 120 g (1 mole) de chlorure de thionyle. On maintient sous agitation à 50°C pendant 2 heures, puis évapore, reprend plusieurs fois au chloroforme, évapore et reprend dans 80 ml de chloroforme pour couler sur une suspension de 27,2 g (180 mM) d'adamantanamine, dans 250 ml de chloroforme et 7,1 g (90 mM) de pyridine.

On porte à reflux pendant 4 heures, puis agite à température ordinaire pendant 15 heures, réfrigère aux environs de 0°C, filtre, évapore à sec le filtrat, reprend à l'acide chlorhydrique 0,5 N glacé (200 ml), extrait au benzène, lave avec 150 ml d'acide chlorhydrique 0,5 N, 2×150 ml d'eau, 2×125 ml de bicarbonate de sodium 1%, 3×150 ml d'eau. Après séchage et évaporation de la couche benzénique, le résidu est recristallisé dans l'alcool isopropylique (4 volumes). On obtient 35% d'un produit pur, Rf = 0,38 (A).

Spectre de RMN en solution dans $CDCl_3$ par rapport au TMS: à 5,9 ppm (s), large, 1 H, NH; 4,5 ppm (m), CH−CONH, 1 H; 2,4 à 2,8 ppm (m), $CH_2-CH_2$ (pyrrolidone), 4 H; 2,5 ppm, (s), $CH_3CO$, 3 H; 1,7 et 2,1 ppm, (2 s), (adamantane), 15 H.

Exemple III

N¹-Cyclopentylpropionyl, N-(Adamantyl-1), (L) Pyroglutamide

1) N¹-Cyclopentylpropionyl (L) pyroglutamate de benzyle

A une suspension de 4,1 g d'hydrure de sodium (85 mM) dans 15 ml de benzène sec, on ajoute en 1 heure une solution de (L) pyroglutamate de benzyle, préparé selon l'exemple II-1), 18,6 g (85 mM), dans 150 ml de benzène. Après agitation pendant 1 heure à température ordinaire, on coule une solution de chlorure de l'acide cyclopentylpropionique, 13,7 g (85 mM), dans 150 ml de benzène, on chauffe à 50°C pendant 5 heures, puis on agite 18 heures à température ordinaire, lave à l'eau, 2×100 ml, sèche et évapore. Le produit est purifié par chromatographie sur colonne de silice par élution avec un mélange chloroforme/acétone. On obtient 73% d'un produit pur, huileux, ne présentant qu'un seul spot en CCM: Rf = 0,80 (A).

2) Acide N[1]-cyclopentylpropionyl (L) pyroglutamique.

On hydrogène à pression atmosphérique le produit précédemment obtenu, 21,3 g (62 mM), dans 220 ml d'éthanol, en présence de 1 g de palladium à 5%/C. Après filtration du catalyseur, on évapore à sec, reprend plusieurs fois au benzène et sèche sous vide poussé (0,05 mmHg) pendant 24 heures. On obtient, avec un rendement de 93%, un produit pur, Rf = 0,50 (A), pF = 135–138°C.

3) N[1]-cyclopentylpropionyl N-(adamantyl-1) (L) pyroglutamide.

On coule l'acide précédemment obtenu, 11,38 g (45 mM), en solution dans 30 ml de chloroforme, sur 65 g (0,55 mole) de chlorure de thionyle. On maintient sous agitation, à 50°C, pendant 2 heures, évapore à sec, reprend plusieurs fois au chloroforme, évapore, et reprend dans 50 ml de chloroforme pour couler sur une suspension de 13,5 g (90 mM) d'adamantanamine, dans 120 ml de chloroforme, et 3,55 g (45 mM) de pyridine. On porte à reflux pendant 4 heures, puis agite à température ordinaire pendant 15 heures, réfrigère aux environs de 0°, filtre et évapore le filtrat, reprend avec 120 ml d'acide chlorhydrique 0,5 N glacé. On extrait au benzène, on lave de façon habituelle (comme dans l'exemple II-4), on sèche et évapore. Le résidu est recristallisé dans l'isopropanol (6 volumes). On obtient 40% d'un produit pur. Rf = 0,53 (A), pF = 169–170°C.

Spectre de RMN en solution dans $CDCl_3$ par rapport au TMS: à 6,1 ppm, (s), large, 1 H, NH; à 4,5 ppm (m), CH CONH, 1 H; 2,4 à 2,8 ppm, (m), $CH_2-CH_2$, pyrrolidone, 4 H; 2,2 ppm, (m),

$$-\underset{\|}{C}-\underline{CH_2}-CH_2-,$$

2 H; 1,7 et 2,1 ppm, (2s), adamantane, 15 H; 1,1 à 1,6 ppm, (m), massif cyclopentyl 11 H.

Exemple IV

N[1]-dipropylacetyl N-[Adamantyl-1] (L) pyroglutamide

1) N[1]-dipropylacétyl (L) pyroglutamate de benzyle.

On dissout à température ordinaire 50,3 g (230 mM) de (L) pyroglutamate de benzyle, préparé selon l'exemple II-1, dans 200 ml de chloroforme et 36,3 g (359 mM) de triéthylamine. On coule une solution de 37,4 g (230 mM) de chlorure de l'acide dipropylacétique dans 50 ml de chloroforme et on porte à reflux pendant 5 heures.

Après refroidissement, on lave à l'eau, sèche, évapore, reprend dans l'hexane et purifie le produit par chromatographie sur colonne d'alumine avec élution à l'hexane. On obtient un produit pur, huileux, Rf = 0,7 (A).

2) Acide N1-dipropylacetyl (L) pyroglutamique.

On hydrogène à pression atmosphérique l'ester précédemment obtenu, 79,4 g (230 mM), dans 800 ml d'alcool isopropylique, en présence de 8 g de palladium à 5%/C. Après filtration du catalyseur, on évapore à sec, reprend plusieurs fois au benzène, évapore à sec. On obtient 95% d'un produit pur, huileux, Rf = 0,50 (A). Il est possible de le convertir en sel de pipérazine (1/1), cristallisé dans un mélange éther éthylique-acétone; pF = 128–130°C, $[\alpha]_D^{22} = -37,2°$ (C = 1, eau).

3) N[1]-dipropylacetyl N-(adamantyl-1) (L) pyroglutamide.

On coule l'acide précédemment obtenu, 11,48 g (45 mM), en solution dans 30 ml de chloroforme, sur 65 g (0,55 mole) de chlorure de thionyle. On maintient sous agitation, à 50°C, pendant 2 heures, évapore à sec, reprend plusieurs fois au benzène, évapore, et reprend dans 50 ml de chloroforme pour couler sur une suspension de 13,6 g (90 mM) d'adamantanamine dans 120 ml de chloroforme et 3,55 g (45 mM) de pyridine. On porte à reflux pendant 5 heures, puis agite à température ambiante pendant 15 heures, réfrigère, filtre et évapore le filtrat. On reprend le résidu avec 120 ml d'acide chlorhydrique 0,5 N glacé, extrait au benzène, lave de façon habituelle (cf. ex II-4), sèche et évapore. Le résidu est recristallisé dans l'isopropanol. On obtient 45% d'un produit pur; Rf = 0,55 (A), Rf = 0,55 (B), pF = 155–157°C.

Spectre de RMN en solution dans $CDCl_3$, par rapport au TMS: à 6,9 ppm, (s), large, NH, 1 H; à 4,6 ppm, (m), CH−CONH, 1 H; à 3,7 ppm, (m), CH−CO−, 1 H; à 2,2–2,7 ppm, (m), $CH_2-CH_2$, pyrrolidone, 4 H; à 1,7 et 2,1 ppm, (2s), adamantane, 15 H; 0,8 à 1,8 ppm, (m), $CH_2$, $CH_3$, 14 H.

Exemple V

N[1]-[Ethyl-2 Hexanoyl] N-[Adamantyl-1] (L) Pyroglutamide

1) N[1]-[éthyl-2 hexanoyl] (L) pyroglutamate de benzyle.

On dissout à température ordinaire 39,4 g (180 mM) de (L) pyroglutamate de benzyle, préparé selon l'exemple II-1, dans 160 ml de chloroforme et 28,4 g (281 mM) de triéthylamine. On coule une solution de 29,3 g (180 mM) du chlorure de l'acide éthyl-2 hexanoïque, dans 40 ml de chloroforme, on porte à reflux pendant 5 heures.

Après refroidissement, on lave à l'eau, sèche, évapore et purifie par chromatographie sur colonne de silice avec élution au benzène. On obtient 80% d'un produit pur, huileux, Rf = 0,70 (A).

2) Acide N[1]-[éthyl-2-hexanoyl] (L) pyroglutamique.

On hydrogène à pression atmosphérique l'ester précédemment obtenu, 48,3 g (140 mM), dans 500 ml d'alcool isopropylique, en présence de 5 g de palladium à 5%/C. Après filtration du catalyseur, on évapore à sec, reprend plusieurs fois au benzène, évapore à sec. On obtient 95% d'un produit pur, huileux, Rf = 0,50 (A). Le produit peut être converti en sel de pipérazine (1/1), par dissolution équimoléculaire dans l'éther (650 ml/100 mM) à reflux et cristallisation à froid: pF = 110–112°C; $[\alpha]_D^{22} = -44,5°$. (C = 1; eau).

3) N1-(éthyl-2-hexanoyl) N-(adamantyl-1) (L) pyroglutamide.

On coule l'acide précédemment obtenu,

16,06 g (63 mM) en solution dans 40 ml de chloroforme, sur 91 g (765 mM) de chlorure de thionyle. On maintient sous agitation, à 50°C, pendant 2 heures, évapore à sec, reprend plusieurs fois au benzène, évapore, et reprend dans 70 ml de chloroforme pour couler sur une suspension de 19,1 g (126 mM) d'adamantanamine, dans 170 ml de chloroforme et 4,98 g (63 mM) de pyridine. On porte à reflux pendant 5 heures, puis on agite à température ambiante pendant 15 heures, réfrigère, filtre et évapore le filtrat. Le résidu est repris dans 170 ml d'acide chlorhydrique 0,5 N glacé, extrait au benzène, lavé de façon habituelle (cf. ex. II-4), séché. Après évaporation, le produit obtenu est recristallisé dans l'isopropanol. On obtient 40% d'un produit pur, Rf = 0,55 (A), Rf = 0,55 (B), pF = 150-152°C. Spectre de RMN en solution dans $CDCl_3$, par rapport au TMS: identique à celui détaillé pour le produit précédent, exemple IV-3.

Exemple VI

$N^1$-Hexanoyl N-(Adamantyl-1) (L) Pyroglutamide

1) $N^1$-hexanoyl (L) pyroglutamate de benzyle.

A une suspension de 3,85 g (80 mM) d'hydrure de sodium, dans 15 ml de benzène sec, on ajoute en 1 heure une solution de (L) pyroglutamate de benzyle, préparé selon l'exemple II-1, 17,5 g (80 mM), dans 150 ml de benzène. Après agitation pendant 1 heure à température ordinaire, on coule une solution de chlorure de l'acide hexanoïque, 10,9 g (81 mM), dans 140 ml de benzène. On chauffe à 50°C pendant 5 heures, puis agite à température ordinaire pendant 15 heures, lave à l'eau (2 × 100 ml), sèche et évapore.

Le résidu est purifié par chromatographie sur colonne de silice, avec élution au chloroforme. On obtient 62% d'un produit huileux, pur, Rf = 0,71 (A), Rf = 0,90 (B).

2) Acide N1-hexanoyl (L) pyroglutamique.

On hydrogène le produit précédemment obtenu, 14,3 g (45 mM) dans 150 ml d'éthanol, en présence de 1 g de palladium à 5%/C. Après filtration du catalyseur, évaporation, et reprise plusieurs fois au benzène, le produit est séché sous vide poussé (0,05 mmHg) pendant 24 heures. On obtient, avec un rendement de 92%, un produit pur, Rf = 0,48 (A), pF = 85-90°C.

3) $N^1$-Hexanoyl N-(adamantyl-1) (L) pyroglutamide.

On coule l'acide précédemment obtenu, 9,08 g (40 mM), en solution dans 30 ml de chloroforme, sur 58,3 g (0,49 mole) de chlorure de thionyle. On maintient sous agitation, à 50°C pendant 2 heures, évapore à sec, reprend plusieurs fois au benzène, évapore et reprend dans 50 ml de chloroforme pour couler sur une suspension de 12,10 g (80 mM) d'adamantanamine, dans 110 ml de chloroforme et 3,17 g (40 mM) de pyridine. On porte à reflux pendant 5 heures, puis agite à température ordinaire pendant 15 heures, réfrigère aux environs de 0°C, filtre, évapore le filtrat. Le résidu est repris dans 110 ml d'acide chlorhydrique 0,5 N glacé, extrait au benzène, lavé de façon habituelle (cf. ex. II-4), séché. Après évaporation, le résidu est recristallisé dans l'alcool isopropylique. On obtient 65% d'un produit pur, Rf = 0,52 (A); pF = 132-134°C.

Spectre de RMN en solution dans $CDCl_3$, par rapport au TMS: à 6,0 ppm (s), large, 1 H, NH; à 4,6 ppm, (m), CHCONH, 1 H; 2,4 à 2,8 ppm (m), $CH_2-CH_2$ (pyrrolidone) et $-CH_2CON$, 6 H; 1,7 à 2,1 ppm (m), adamantane, 15 H; 1,3 à 1,7 ppm, (m), $-CH_2-CH_2-CH_2-CH_3$, 6 H; 0,85 ppm, (t), $CH_3-CH_2-$, 3 H.

Exemple VII

$N^1$-Benzoyl N-(Adamantyl-1) (L) Pyroglutamide

1) $N^1$-benzoyl (L) pyroglutamate de benzyle.

A une suspension de 4,8 g (0,1 mole) d'hydrure de sodium dans 15 ml de benzène sec, on ajoute en une heure 21,9 g (0,1 mole) de (L) pyroglutamate de benzyle (préparé selon l'exemple II-1), en solution dans 150 ml de benzène. On agite pendant une heure à température ordinaire puis introduit 14,1 g (0,1 mole) de chlorure de benzoyle dilué dans le benzène. On agite le mélange réactionnel pendant 4 heures à 55°C puis pendant 18 heures à température ordinaire. Après lavage à l'eau, évaporation du solvant et reprise à l'éther, on obtient des cristaux blancs de pF = 101/102°C; Rdt = 70%; Rf = 0,68 (A).

2) Acide $N^1$-benzoyl (L) pyroglutamique.

On hydrogène à pression atmosphérique l'ester précédemment obtenu 19,4 g (60 mM) à 40°C, dans 350 ml d'éthanol, en présence de 2 g de palladium à 5%/C. Après filtration du catalyseur, on évapore à sec. Le résidu est recristallisé dans 7 à 8 volumes d'éthanol. On obtient 87% d'un produit pur; Rf = 0,53 (A) - pF = 140-141°C.

3) $N_1$-benzoyl N-(adamantyl-1) (L) pyroglutamide.

On disperse 11,65 g (50 mM) de l'acide précédemment obtenu dans 77 g (0,65 mole) de chlorure de thionyle, on solubilise à 40°C, agite pendant 1 heure, évapore, reprend avec de l'éther sec. On obtient après filtration une poudre de couleur crème, avec un rendement de 81%. A une suspension de 12,1 g (80 mM) d'adamantanamine, dans 130 ml de chloroforme et 3,17 g (40 mM) de pyridine, on ajoute une solution de chlorure d'acide précédemment obtenu, 10,1 g (40 mM), dans 180 ml de chloroforme. On porte à reflux pendant 3 heures, agite à température ordinaire pendant 15 heures, réfrigère, filtre, puis après les lavages habituels (cf. ex. II-4), on sèche et évapore. Le résidu est trituré dans l'éther, filtré, puis recristallisé dans l'alcool isopropylique (13 volumes). On obtient 52% d'un produit pur; Rf = 0,60 (A); Rf = 0,32 (B); pF = 196-197°C; $|\alpha|_D$ = −4,4° (C = 1, $CHCl_3$).

Spectre de RMN en solution dans $CDCl_3$ par rapport au TMS: 7,25 à 7,70 ppm, (m), $C_6H_5CO$, 5 H; 5,85 ppm, (s), large, NH, 1 H; 4,60 ppm, (m), CHCONH, 1 H; 2,40 à 2,85 ppm (m), $CH_2-CH_2$, pyrrolidone, 4 H; 1,7 et 2,05 ppm, (2s), adamantane, 15 H.

Exemple VIII

$N^1$-Paramethoxybenzoyl N-(Adamantyl-1) (L) Pyroglutamide

1) $N^1$-paraméthoxybenzoyl (L) pyroglutamate de benzyle.

A une suspension de 4,08 g (85 mM) d'hydrure de sodium dans 15 ml de benzène sec, on ajoute en 1 heure une solution de (L) pyroglutamate de benzyle, préparé selon l'exemple II-1), 18,6 g (85 mM), dans 150 ml de benzène. Après agitation pendant 1 heure à température ordinaire, on coule une solution du chlorure de l'acide p. méthoxy benzoïque, 14,7 g (86 mM), dans 150 ml de benzène. On chauffe à 50°C pendant 5 heures, on agite à température ordinaire pendant 18 heures, lave à l'eau (2 × 100 ml), sèche et évapore. Le résidu est purifié par chromatographie sur colonne de silice, avec élution par un mélange de chlorure de méthylène et d'éther (0,5%).

On obtient 50% d'un produit pur, huileux, Rf = 0,68 (A), Rf = 0,90 (B).

2) Acide $N^1$-p-méthoxybenzoyl (L) pyroglutamique.

On hydrogène le produit précédemment obtenu, 14,1 g (40 mM), dans 200 ml d'éthanol, en présence de 1,2 g de palladium à 5%/C, à 40°. Après filtration du catalyseur, évaporation, le résidu est trituré à l'éther, l'éther évaporé. On obtient ainsi 93% d'un produit pur, Rf = 0,52 (A), pF = 149–150°C. $[\alpha]_D$ = +59,5° (C = 1, DMF).

3) $N_1$-p-methoxybenzoyl N-(adamantyl-1) (L) pyroglutamide.

On coule l'acide précédemment obtenu, 9,2 g (35 mM), en solution dans 10 ml de chloroforme, sur 51,2 g (0,43 mole) de chlorure de thionyle; on maintient sous agitation, à 50°C, pendant 2 heures, évapore à sec, reprend plusieurs fois au benzène, évapore et reprend dans 50 ml de chloroforme pour couleur sur une suspension de 10,6 g (70 mM) d'adamantanamine, dans 100 ml de chloroforme et 2,77 g (35 mM) de pyridine. On maintient à reflux pendant 4 heures, puis à température ordinaire pendant 15 heures, réfrigère, filtre, évapore le filtrat. Le résidu est repris par 140 ml d'acide chlorhydrique 0,5 N glacé et extrait au benzène. Après les lavages classiques (ex. II-4) de la couche benzénique, on sèche et recristallise le résidu d'évaporation dans un minimum d'alcool isopropylique: on obtient ainsi 41% d'un produit pur. Rf = 0,42 (A), pF = 197–199°C.

Spectre de RMN, en solution dans $CDCl_3$, par rapport au TMS: 7,65 ppm, (d), H arom ortho de $-CON$, 2 H; 6,90 ppm, (d), H arom ortho de $OCH_3$, 2 H; 5,70 ppm (s), large, NH, 1 H; 4,55 ppm, (m), CHCONH, 1 H; 3,80 ppm, (s), $OCH_3$, 3 H; 2,40 à 2,85 ppm, $CH_2CH_2$, pyrrolidone, 4 H; 1,65 et 2,05 ppm (2s), adamantane, 15 H.

Exemple IX

$N^1$-[Dimethoxy-3,4 Benzoyl] N-[Adamantyl-1] (L) Pyroglutamide

1) $N^1$-(diméthoxy-3,4, benzoyl) (L) pyroglutamate de benzyle.

A une suspension de 5,28 g (0,11 mole) d'hydrure de sodium, dans 20 ml de benzène sec, on ajoute en 1½ heure une solution de (L) pyroglutamate de benzyle, 24,1 g (0,11 mole), préparé selon l'exemple II-1), dans 200 ml de benzène. Après agitation pendant 1 heure à température ordinaire, on coule une solution de chlorure de l'acide diméthoxy-3,4 benzoïque, 22,1 g (0,11 mole), dans 180 ml de benzène.

On chauffe à 55°C pendant 5 heures, puis on agite à température ordinaire pendant 18 heures, lave à l'eau (2 × 150 ml), sèche, évapore. Le résidu est purifié par chromatographie sur colonne de silice, avec élution par un mélange chlorure de méthylène-éther. On obtient 40% d'un produit pur, Rf = 0,65 (A); Rf = 0,60 (B).

2) Acide $N^1$-(diméthoxy-3,4 benzoyl) (L) pyroglutamique.

On hydrogène à pression atmosphérique l'ester précédemment obtenu, 15,3 g (40 mM), dans 250 ml d'éthanol, à 45°C, en présence de 1,5 g de palladium à 5%/C. Après filtration du catalyseur, évaporation du filtrat, reprise du résidu au benzène, évaporation, le produit est trituré à l'éther, puis filtré. On obtient 75% d'un produit pur, pF = 152–153°C, Rf = 0,28 (A).

3 $N^1$-(diméthoxy-3,4 benzoyl) N-(adamantyl-1) (L) pyroglutamide.

On ajoute en portions l'acide précédemment obtenu, 8,8 g (30 mM), sur 44 g (0,37 mole) de chlorure de thionyle. On agite pendant 2 heures à 50°C, évapore à sec, reprend plusieurs fois au benzène, évapore, reprend dans 70 ml de chloroforme pour couler sur une suspension de 9,08 g (60 mM) d'adamantanamine, dans 80 ml de chloroforme et 2,4 g (30 mM) de pyridine. On porte à reflux pendant 4 heures, agite à température ordinaire pendant 15 heures, réfrigère, filtre et évapore le filtrat. Le résidu est repris dans l'acide chlorhydrique 0,5 N, extrait et lavé de façon habituelle (cf. ex. II-4). Après évaporation de la couche benzénique, le produit est purifié par chromatographie sur colonne de silice avec élution par un mélange chloroforme-acétone, puis recristallisation dans un mélange alcool isopropylique-éther. On obtient 35% d;un produit pur; Rf = 0,33 (A); Rf = 0,18 (B); pF = 205–207°C.

Spectre de RMN en solution dans $CDCl_3$ par rapport au TMS: 6,90 à 7,40 ppm, (m), H arom, 3 H; 5,75 ppm, (s), large, NH, 1 H; 4,50 ppm, (m), CHCONH, 1 H; 3,80 et 3,85 ppm, (2s), $OCH_3$, 6 H; 2,20 et 2,70 ppm, (m), $CH_2-CH_2$, pyrrolidone; 1,65 et 2,05 ppm, (2s), adamantane,15 H.

Exemple X

$N^1$-[Phenylacetyl] N-[Adamantyl-1] (L) Pyroglutamide

1) $N^1$[phényl acétyl] (L) pyroglutamate de benzyle.

A une suspension de 4,1 g (85 mM) d'hydrure de sodium dans 15 ml de benzène sec, on ajoute en 1 heure une solution de (L) pyroglutamate de benzyle, 18,6 g (85 mM), préparé selon l'exemple II-1), dans 150 ml de benzène.

Après agitation pendant 1 heure à température ordinaire, on coule une solution du chlorure de l'acide phényl acétique, 13,3 g (86 mM), dans 150

ml de benzène. On chauffe pendant 5 heures à 55°C, puis agite 15 heures à température ordinaire, lave à l'eau (2 × 120 ml), sèche, évapore. Le résidu est purifié par chromatographie sur colonne de silice, avec élution au chloroforme. On obtient 58% d'un produit pur, huileux, Rf = 0,72 (A), Rf = 0,90 (B).

2) Acide N[1]-phénylacétyl (L) pyroglutamique.

On hydrogène à pression atmosphérique l'ester obtenu précédemment, 15,2 g (45 mM), dans 200 ml d'éthanol, à température ordinaire, en présence de 1,5 g de palladium à 5%/C. Après filtration du catalyseur, on évapore à sec le filtrat, reprend plusieurs fois au benzène, évapore et sèche sous vide poussé (0,05 mmHg). On obtient ainsi 95% d'un produit pur, cireux, Rf = 0,42 (A).

3) N[1]-(phényl acétyl) N-(adamantyl-1) (L) pyroglutamide.

On coule l'acide précédemment obtenu, 9,9 g (40 mM), en solution dans 40 ml de chloroforme, sur 59 g (0,49 mole) de chlorure de thionyle. On maintient sous agitation, à 50°C, pendant 2 heures, évapore à sec, reprend plusieurs fois au benzène, évapore, et reprend dans 60 ml de chloroforme pour couler sur une suspension de 12,1 g (80 mM) d'adamantanamine, dans 100 ml de chloroforme et 3,17 g (40 mM) de pyridine. On porte à reflux pendant 5 heures, puis agite à température ordinaire pendant 15 heures, réfrigère, filtre et évapore le filtrat. Le résidu est repris à l'acide chlorhydrique 0,5 N, extrait et lavé de façon habituelle (cf. ex. II-4). Après séchage et évaporation, le résidu est purifié par chromatographie sur colonne de silice, avec élution par un mélange chloroforme-acétone. On obtient ainsi 45% d'un produit pur, Rf = 0,50 (A); Rf = 0,45 (B); pF = 130–132°C.

Spectre de RMN en solution dans $CDCl_3$ par rapport au TMS: 7,25 ppm, (s), $C_6H_5$, 5 H; 5,80 ppm, (s), large, NH, 1 H; 4,50 ppm, (m), CHCONH, 1 H; 2,40 à 2,90 ppm, (m), $-CH_2-CH_2-$, pyrrolidone, 4 H; 1,70 et 2,0 ppm, (2s), adamantane, 15 H.

Exemple XI

N[1]-(Dimethoxy-3,4 Phényl Acétyl)N-(Adamantyl-1) (L) Pyroglutamide

1) N[1]-(Diméthoxy-3,4 Phényl Acétyl) (L) Pyroglutamate de benzyle.

A une suspension de 3,85 g (80 mM) d'hydrure de sodium, dans 15 ml de benzène sec, on ajoute en 1 heure une solution de (L) pyroglutamate de benzyle, préparé selon l'exemple II-1), 17,5 g (80 mM) dans 150 ml de benzène. Après agitation pendant 1 heure à température ordinaire, on coule une solution du chlorure de l'acide diméthoxy-3,4 phényl acétique, 17,2 g (80 mM) dans 150 ml de benzène. On chauffe à 55°C pendant 6 heures, agite pendant 15 heures à température ordinaire, lave avec 2 × 120 ml, sèche, évapore. Le résidu est purifié par chromatographie sur colonne de silice, avec élution par un mélange chloroforme-acétone. On obtient ainsi 60% d'un produit pur, cireux, Rf = 0,46 (A).

2) Acide N[1]-diméthoxy-3,4 phényl acétyl (L) pyroglutamique.

On hydrogène à température ordinaire et pression atmosphérique l'acide précédemment obtenu, 19,9 g (50 mM), dans 300 ml d'éthanol, en présence de 2 g de palladium à 5%/C.

Après filtration du catalyseur, le filtrat est évaporé, repris plusieurs fois au benzène, évaporé à sec et séché sous vide poussé (0,05 mmHg). On obtient ainsi 93% d'un produit pur, cireux, Rf = 0,33 (A).

3) N[1]-(diméthoxy-3,4 phényl acétyl) N-adamantyl-1) (L) pyroglutamide.

On coule l'acide précédemment obtenu, 13,8 g (45 mM), en solution dans 35 ml de chloroforme, sur 65,5 g (550 mM) de chlorure de thionyle; on maintient à 50°C pendant 2 heures, évapore à sec, reprend plusieurs fois au benzène, évapore à sec, et reprend avec 50 ml de chloroforme pour couler sur une suspension de 13,6 g (90 mM) d'adamantanamine, dans 120 ml de chloroforme et 3,6 g (45 mM) de pyridine. On porte à reflux pendant 6 heures, agite à température ordinaire pendant 15 heures, réfrigère, filtre et évapore le filtrat. Le résidu est traité de façon habituelle (cf. exemple II-4). Après évaporation de la couche benzénique, le produit est purifié par chromatographie sur colonne de silice, avec élution par un mélange chloroforme-acétone. On obtient ainsi 35% d'un produit pur; Rf = 0,42 (A), Rf = 0,31 (B), pF = 75–77°C.

Spectre de RMN en solution dans $CDCl_3$ par rapport au TMS: 6,80 ppm, (s), $(OCH_3)_2$ $C_6H_3$ $-CH_2$, 3 H; 5,75 ppm, (s) large, NH, 1 H; 4,55 ppm, (m), CHCONH, 1 H; 4,2 ppm, (s), $CH_2CON$, 2 H; 3,85 ppm, (s), $OCH_3 \times 2$,6 H; 2,40 à 2,85 ppm, (m), $-CH_2CH_2-$, pyrrolidone, 4 H; 1,60 et 1,95 ppm, (2s), adamantane, 15 H.

Exemple XII

N[1][Methoxy-2 Ethyl], N-[Adamantyl-1] (L) Pyroglutamide

1) N1(méthoxy-2) (L) pyroglutamate de benzyle.

A une suspension de 6,24 g (0,13 mole) d'hydrure de sodium dans 30 ml de N,N diméthylformamide, on ajoute en 1,5 heure une solution de (L) pyroglutamate de benzyle, préparé selon l'exemple II-1, 28,5 g (0,13 mole), dans 200 ml de N,N diméthylformamide. On agite pendant 1 heure à température ordinaire, puis on coule une solution du chlorure de méthoxy-2-éthyl, 14,7 g (156 mM), dans 100 ml de diméthylformamide. On chauffe à 70°C pendant 5 heures, agite pendant 15 heures à température ordinaire, évapore à sec, reprend par 200 ml d'acide chlorhydrique 0,5 N, extrait au benzène, lave à l'eau, et sèche. Après évaporation de la couche benzénique, le résidu est purifié par chromatographie sur colonne de silice, avec élution par un mélange chlorure de méthylène-éther. On obtient 45% d'un produit pur, Rf = 0,45 (A).

2) Acide N1-(méthoxy-2 éthyl) (L) pyroglutamique.

On hydrogène à température ordinaire et pression atmosphérique l'ester précédemment obtenu, 15,2 g (55 mM), dans 200 ml d'éthanol, en présence de 1,5 g de palladium à 5%/C. Après filtration du catalyseur, évaporation du filtrat, et reprise plusieurs fois au benzène, le résidu est séché sous vide poussé (0,05 mmHg) pendant 18 heures. On obtient ainsi 97% d'un produit pur, Rf = 0,18 (A), pF = 96–97 °C.

3) $N^1$-(méthoxy-2 éthyl) N-(adamantyl-1) (L) pyroglutamide.

On ajoute en portions 9,35 g (50 mM), de l'acide précédemment obtenu à 72 g (0,6 mole) de chlorure de thionyle. On maintient à 50 °C pendant 2 heures, évapore et reprend plusieurs fois au benzène, évapore, et reprend le résidu dans 50 ml de chloroforme pour couler sur une suspension de 15,1 g (0,1 mole) d'adamantanamine, dans 120 ml de chloroforme et 3,95 g (50 mM) de pyridine. On chauffe à reflux pendant 5 heures, puis agite à température ordinaire pendant 15 heures, réfrigère, filtre, évapore le filtrat. Le résidu est traité de façon habituelle (cf. ex. II-4). Le produit est purifié par chromatographie sur colonne de silice, avec élution par un mélange chloroforme-acétone. On obtient ainsi 53% d'un produit pur; Rf = 0,22 (A); Rf = 0,10 (B); pF = 128–129 °C.

Spectre de RMN en solution dans $CDCl_3$ par rapport au TMS: 6,15 ppm (s) large, NH, 1 H; 4,10 ppm, (m), CHCONH, 1 H; 3,3 à 3,7 ppm, (m), $N-CH_2-CH_2-O-$, 4 H; 3,4 ppm, (s), $OCH_3$, 3 H; 2,15 à 2,65 ppm, (m), $-CH_2-CH_2-$, pyrrolidone, 4 H; 1,75 et 2,05 ppm, (2s), adamantane, 15 H.

Exemple XIII

$N^1$-[Cyano-2 Ethyl] N-[Adamantyl-1] (L) Pyroglutamide

On ajoute en portions 10,92 g (60 mM) de l'acide $N^1$-]cyano-2 éthyl] (L) pyroglutamique à 86 g (0.72 Mole) de chlorure de thionyle, dans 60 ml de chloroforme. On maintient au reflux du chloroforme pendant 2 heures, évapore à sec, reprend plusieurs fois au benzène, évapore et reprend le résidu dans 50 ml de chloroforme pour couler sur une suspension de 18,15 g (120 mM) d'adamantanamine, dans 250 ml de chloroforme et 4,9 g (60 mM) de pyridine. On porte au reflux pendant 4 heures, agite une nuit à température ordinaire, réfrigère, filtre et évapore le filtrat. Le résidu est traité de façon habituelle, (cf. exemple II-4); on purifie le produit brut par recristallisation dans un minimum d'alcool isopropylique. On obtient 30% d'un produit pur, Rf = 0,24 (A), Rf = 0,14 (B); pF = 170–172 °C.

Spectre de RMN, en solution dans $CDCl_3$ par rapport au TMS: 6,20 ppm, (s), NH, 1 H; 4,15 ppm, (m), CHCONH, 1 H; 3,75 ppm, (m), $N-CH_2-CH_2-$, 2 H; 3,30 ppm, (m), $-CH_2-CH_2-$ CN, 2 H; 2,40 à 2,85 ppm, (m), $-CH_2-CH_2$, pyrrolidone, 4 H; 1,70 et 2,0 ppm, (2s), adamantane, 15 H.

La transformation des dérivés selon l'invention en des sels pharmacologiquement acceptables peut être effectuée selon les techniques habituelles.

Les propriétés antivirales des dérivés peuvent être mises en évidence selon les techniques connues. Ainsi par administration à des souris par voie intrapéritonéale ou orale des dérivés selon l'invention à doses répétées toutes les 6 heures, comprises entre 2 et 40 mg/kg de poids et commencées 30 minutes avant l'infection expérimentale par influenzae A.Z. intranasal, on note une excellente activité antivirale. Pour des niveaux d'infection moyens, la protection se manifeste par l'augmentation du nombre de survies et l'accroissement de la durée de survie. Pour des infections à haute dose, près de 20 fois la DL 50, le nombre de survie n'est pas augmenté mais l'on note un allongement significatif du temps de survie. Les meilleurs résultats sont obtenus si l'on administre les produits plus de quatre heures avant l'infection expérimentale ce qui démontre l'intérêt d'un usage prophylactique de tels composés vis-à-vis des infections virales du type influenzae.

Les dérivés selon l'invention se révèlent également hypocholestérolémiants et hypobétalipoprotéinémiants.

Leur administration à des doses comprises entre 0,1 et 0,7 mMole/kg à des rats hypocholestérolémiques, entraîne une baisse de la concentration en cholestérol sérique d'environ 20% et une réduction de la valeur du rapport H.P.L. («Heparine precipitating lipoproteins»)/cholestérol jusqu'à une valeur moyenne de 0,91.

Les dérivés selon l'invention possèdent d'autre part des propriétés anticonvulsivantes, ils retardent la crise convulsive au curare et sont antianoxiques dans le test de confinement à des doses voisines de 100 mg/kg par voie orale. Ils manifestent également une bonne activité anticataleptique à ces mêmes doses.

Les dérivés selon la formule générale (I) sont administrés de préférence par voie orale, mais ils peuvent selon les besoins être administrés par les autres voies: parentérale, sous-cutanée, intraveineuse, intramusculaire, intrapéritonéale ou transcutanée.

Cette administration peut être faite également en combinaison avec des excipients ou diluants pharmaceutiquement acceptables.

**Revendications pour les états contractants: BE, CH, DE, IT, LI, LU, NL, SE**

1. Dérivés du N-(adamantyl-1) pyrrolidone-5 carboxamide-2 répondant à la formule générale I:

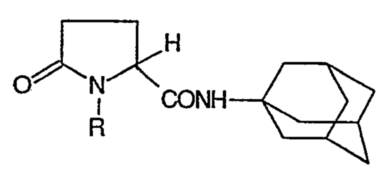

(I)

où

R représente l'hydrogène, ou un radical alkyle linéaire ou ramifié comprenant de 1 à 7 atomes de carbones substitué ou non par un groupement alkoxyle, thioalkyle ou nitrile,

– ou des radicaux acyles du type

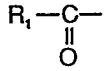 ou 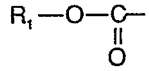

avec $R_1$ représentant:
- un radical alkyle linéaire ou ramifié comprenant de 1 à 7 atomes de carbone,
- ou un radical cycloalkyle comprenant de 5 à 7 atomes de carbone, le radical cycloalkyle pouvant être relié au radical carbonale par un ou deux groupements méthylène,
- ou un noyau aromatique, éventuellement substitué par des substituants tels que le groupe nitro, alkoxyle de $C_1$ à $C_3$, alkyle de $C_1$ à $C_3$ ou l'atome d'halogène,
- un radical benzylique éventuellement substitué par des substituants tels que l'atome d'halogène, ou les groupes nitro, alkoxyle ou alkyle de $C_1$ à $C_3$.

2. Composé selon la revendication 1, caractérisé en ce que le radical pyrrolidone carboxamide est de configuration DL.

3. Composé selon la revendication 1 caractérisé en ce que le radical pyrrolidone carboxamide est de configuration L.

4. Composé selon la revendication 1 où R représente le radical diméthoxy-3,4 benzoyle.

5. Composé selon la revendication 1 caractérisé en ce que R = H.

6. L'acide N[1]-diméthoxy-3,4 benzoyl pyroglutamique à titre du produit industriel nouveau, intermédiaire de synthèse du dérivé selon la revendication 4.

7. Composé selon la revendication 1 où R représente le radical méthoxy-4 benzoyle.

8. L'acide N[1]-méthoxy-4 benzoyl pyroglutamique à titre de produit industriel nouveau, intermédiaire de fabrication du dérivé selon la revendication 7.

9. Composé selon la revendication 1 où R représente le radical, diméthoxy-3,4 phényl acétyle.

10. L'acide N[1]-diméthoxy-3,4 phényl acétylpyroglutamique à titre de produit industriel nouveau, intermédiaire de fabrication du dérivé selon la revendication 9.

11. Composé selon la revendication 1 où R représente le radical cyclopentylpropionyle.

12. L'acide N[1]-cyclopentylpropionyl pyroglutamique à titre de produit industriel nouveau, intermédiaire de fabrication du dérivé selon la revendication 11.

13. Composé selon la revendication 1 où R représente le radical propyl-2 pentanoyle.

14. L'acide N[1]-(propyl)-2 pentanoyl pyroglutamique à titre de produit industriel nouveau, intermédiaire de fabrication du dérivé selon la revendication 13.

15. Composé selon la revendication 1 où R représente le radical éthyl-2 hexanoyle.

16. L'acide N[1]-(éthyl)-2 hexanoyl-pyroglutamique à titre de produit industriel nouveau, intermédiaire de fabrication du dérivé selon la revendication 15.

17. Composé selon la revendication 1 où R représente le radical hexanoyle.

18. L'acide N[1]-hexanoyl pyroglutamique à titre de produit industriel nouveau, intermédiaire de fabrication du dérivé selon la revendication 17.

19. Médicaments constitués par ou contenant au moins un composé selon la revendication 1.

20. Procédé de préparation des composés selon la revendication 1 caractérisé en ce que l'on fait réagir le dérivé sodé de l'ester benzylique de l'acide pyroglutamique avec un agent d'alkylation ou d'acylation pour obtenir le dérivé N[1]-alkyle ou N[1]-acyle du pyroglutamate de benzyle, lequel composé après hydrogénolyse est mis en réaction avec l'adamantanamine en présence d'un agent de couplage dans un solvant inerte.

21. Procédé suivant la revendication 20 où l'acide N[1]-alkyl ou N[1]-acylpyroglutamique est transformé en un dérivé réactif qui est mis ensuite en réaction avec l'adamantanamine dans un solvant inerte avec éventuellement un accepteur d'hydrogène.

22. Procédé de préparation du composé selon la revendication 5, caractérisé en ce que l'on prépare un dérivé réactif de l'acide N[1]-benzyloxycarbonyl-pyroglutamique que l'on fait réagir sur l'adamantanamine, et que l'on hydrogénolyse pour obtenir le composé recherché.

**Revendications pour l'état contractant: AT**

1. Procédé de préparation de dérivés du N-(adamantyl-1) pyrrolidone-5 carboxamide-2 répondant à la formule générale I:

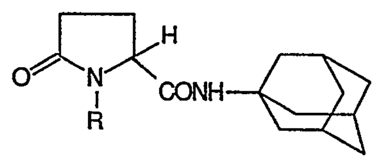 (I)

où
R représente l'hydrogène, ou un radical alkyle linéaire ou ramifié comprenant de 1 à 7 atomes de carbone substitué ou non par un groupement alkoxyle, thioalkyle ou nitrile,
- ou des radicaux acyles du type

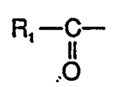 ou 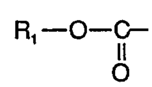

avec $R_1$ représentant:
- un radical alkyle linéaire ou ramifié comprenant de 1 à 7 atomes de carbone,
- ou un radical cycloalkyle comprenant de 5 à 7 atomes de carbone, le radical cycloalkyle pouvant être relié au radical carbonyle par un ou deux groupements méthylène,
- ou un noyau aromatique, éventuellement substitué par des substituants tels que le groupe nitro, alkoxyle de $C_1$ à $C_3$, alkyle de $C_1$ à $C_3$ ou l'atome d'halogène,
- un radical benzylique éventuellement substitué par des substituants tels que l'atome d'halogène, ou les groupes nitro, alkoxyle ou alkyle de $C_1$ à $C_3$, caractérisé en ce que l'on fait réagir le dérivé

sodé de l'ester benzylique de l'acide pyroglutamique avec un agent d'alkylation ou d'acylation pour obtenir le dérivé $N^1$-alkyle ou $N^1$-acyle du pyroglutamate de benzyle, lequel composé après hydrogénolyse est mis en réaction avec l'adamantanamine en présence d'un agent de couplage dans un solvant inerte.

2. Procédé selon la revendication 1 où l'acide $N^1$-alkyl ou $N^1$-acylpyroglutamique est transformé en un dérivé réactif qui est mis ensuite en réaction avec l'adamantanamine dans un solvant inerte avec éventuellement un accepteur d'hydrogène.

3. Procédé selon la revendication 1 ou la revendication 2, caractérisé en ce que le radical pyrrolidone carboxamide est de configuration DL.

4. Procédé selon la revendication 1 ou la revendication 2, caractérisé en ce que le radical pyrrolidone carboxamide est de configuration L.

**Claims for the Contracting States: BE, CH, DE, IT, LI, LU, NL, SE**

1. An N-(adamant-1-yl)pyrrolid-5-one-2-carboxamide derivative of general formula I

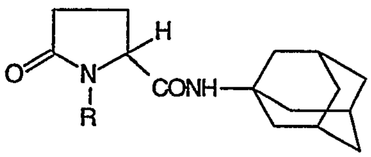

(I)

in which
R represents hydrogen or a linear or branched alkyl radical containing from 1 to 7 carbon atoms which is unsubstituted or substituted by an alkoxy, thioalkyl or nitrile group,
- or an acyl radical of the type

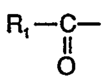 or 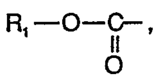,

where $R_1$ represents:
- a linear or branched alkyl radical containing from 1 to 7 carbon atoms,
- or a cycloalkyl radical containing from 5 to 7 carbon atoms, it being possible for the cycloalkyl radical to be joined to the carbonyl radical by one or two methylene groups,
- or an aromatic ring which is unsubstituted or substituted by substituents such as the nitro group, a $C_1$ to $C_3$ alkoxy group, a $C_1$ to $C_3$ alkyl or a halogen atom,
- or a benzyl radical which is unsubstituted or substituted by substituents such as a halogen atom, the nitro group or a $C_1$ to $C_3$ alkoxy or alkyl group.

2. A compound according to Claim 1, characterized in that the pyrrolidonecarboxamide radical has the DL configuration.

3. A compound according to Claim 1, characterized in that the pyrrolidonecarboxamide radical has the L configuration.

4. The compound according to Claim 1 in which R represents the 3,4-dimethoxybenzoyl radical.

5. The compound according to Claim 1 in which R = H.

6. $N^1$-(3,4-Dimethoxybenzoyl)pyroglutamic acid as a novel industrial product which is an intermediate in the synthesis of the derivative according to Claim 4.

7. The compound according to Claim 1 in which R represents the 4-methoxybenzoyl radical.

8. $N^1$-(4-Methoxybenzoyl)pyroglutamic acid as a novel industrial product which is an intermediate in the manufacture of the derivative according to Claim 7.

9. The compound according to Claim 1 in which R represents the 3,4-dimethoxyphenylacetyl radical.

10. $N^1$-(3,4-Dimethoxyphenylacetyl)pyroglutamic acid as a novel industrial product which is an intermediate in the manufacture of the derivative according to Claim 9.

11. The compound according to Claim 1 in which R represents the cyclopentylpropionyl radical.

12. $N^1$-(Cyclopentylpropionyl)pyroglutamic acid as a novel industrial product which is an intermediate in the manufacture of the derivative according to Claim 11.

13. The compound according to Claim 1 in which R represents the 2-propylpentanoyl radical.

14. $N^1$-(2-Propylpentanoyl)pyroglutamic acid as a novel industrial product which is an intermediate in the manufacture of the derivative according to Claim 13.

15. The compound according to Claim 1 in which R represents the 2-ethylhexanoyl radical.

16. $N^1$-(2-Ethylhexanoyl)pyroglutamic acid as a novel industrial product which is an intermediate in the manufacture of the derivative according to Claim 15.

17. The compound according to Claim 1 in which R represents the hexanoyl radical.

18. $N^1$-Hexanoylpyroglutamic acid as a novel industrial product which is an intermediate in the manufacture of the derivative according to Claim 17.

19. A drug consisting of or containing at least one compound according to Claim 1.

20. A process for the preparation of the compounds according to Claim 1, characterized in that the sodium derivative of benzyl pyroglutamate is reacted with an alkylating or acylating agent to give the $N^1$-alkyl or $N^1$-acyl derivative of benzyl pyroglutamate, which compound is subjected to hydrogenolysis and then reacted with adamantanamine, in the presence of a coupling agent, in an inert solvent.

21. A process according to Claim 20 in which the $N^1$-alkylpyroglutamic or $N^1$-acylpyroglutamic acid is converted into a reactive derivative, which

is then reacted with adamantanamine in an inert solvent, if appropriate in the presence of a hydrogen acceptor.

22. A process for the preparation of the compound according to Claim 5, characterized in that a reactive derivative of $N^1$-(benzyloxycarbonyl)pyroglutamic acid is prepared and reacted with adamantanamine, and in that the reaction product is subjected to hydrogenolysis to give the desired compound.

**Claims for the Contracting State: AT**

1. A process for the preparation of N-(adamant-1-yl)-pyrrolid-5-one-2-carboxamide derivatives of general formula I

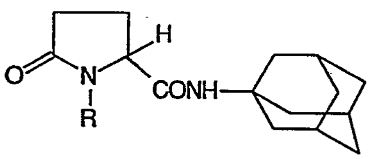

(I)

in which
R represents hydrogen or a linear or branched alkyl radical containing from 1 to 7 carbon atoms which is unsubstituted or substituted by an alkoxy, thioalkyl or nitrile group,
- or an acyl radical of the type

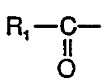 or 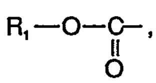

where $R_1$ represents:
- a linear or branched alkyl radical containing from 1 to 7 carbon atoms,
- or a cycloalkyl radical containing from 5 to 7 carbon atoms, it being possible for the cycloalkyl radical to be joined to the carbonyl radical by one or two methylene groups,
- or an aromatic ring which is unsubstituted or substituted by substituents such as the nitro group, a $C_1$ to $C_3$ alkoxy group, a $C_1$ to $C_3$ alkyl group or a halogen atom,
- or a benzyl radical which is unsubstituted or substituted by substituents such as a halogen atom, the nitro group or a $C_1$ to $C_3$ alkoxy or alkyl group, characterized in that the sodium derivative of benzyl pyroglutamate is reacted with an alkylating or acylating agent to give the $N^1$-alkyl or $N^1$-acyl derivative of benzyl pyroglutamate, which compound is subjected to hydrogenolysis and then reacted with adamantanamine, in the presence of a coupling agent, in an inert solvent.

2. A process according to Claim 1 in which the $N^1$-alkylpyroglutamic or $N^1$-acylpyroglutamic acid is converted into a reactive derivative, which is then reacted with adamantanamine in an inert solvent, if appropriate in the presence of a hydrogen acceptor.

3. A process according to Claim 1 or Claim 2, characterized in that the pyrrolidonecarboxamide radical has the DL configuration.

4. A process according to Claim 1 or Claim 2, characterized in that the pyrrolidonecarboxamide radical has the L configuration.

**Patentansprüche für die benannten Vertragsstaaten: BE, CH, LI, DE, IT, LU, NL, SE**

1. N-(1-Adamantyl)-5-pyrrolidon-2-carboxamid-Verbindungen der allgemeinen Formel I

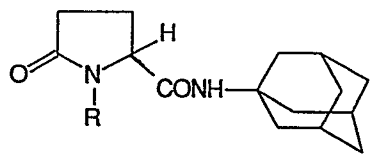

(I)

in der R für Wasserstoff oder eine lineare oder verzweigte Alkylgruppe mit 1 bis 7 Kohlenstoffatomen steht, die gegebenenfalls substituiert ist mit einer Alkoxyl-, Thioalkyl- oder Nitrilgruppe, oder für Acylgruppen vom Typ

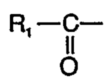 oder 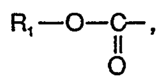

wobei $R_1$ bedeutet:
- eine lineare oder verzweigte Alkylgruppe mit 1 bis 7 Kohlenstoffatomen,
- oder eine Cycloalkylgruppe mit 5 bis 7 Kohlenstoffatomen, die an die Carbonylgruppe über eine oder zwei Methylengruppen gebunden sein kann,
- oder einen aromatischen Kern, gegebenenfalls substituiert mit Substituenten, wie Nitrogruppe, $C_1$- bis $C_3$-Alkoxylgruppe, $C_1$- bis $C_3$-Alkylgruppe oder Halogenatom,
- eine Benzylgruppe, gegebenenfalls substituiert mit Substituenten, wie Halogenatom oder Nitro-, $C_1$- bis $C_3$-Alkoxyl- oder $C_1$- bis $C_3$-Alkylgruppen.

2. Verbindung nach Anspruch 1, dadurch gekennzeichnet, dass die Pyrrolidoncarboxamid-Gruppe die Konfiguration DL aufweist.

3. Verbindung nach Anspruch 1, dadurch gekennzeichnet, dass die Pyrrolidoncarboxyamid-Gruppe die Konfiguration L aufweist.

4. Verbindung nach Anspruch 1, dadurch gekennzeichnet, dass R die 3,4-Dimethoxybenzoylgruppe bedeutet.

5. Verbindung nach Anspruch 1, dadurch gekennzeichnet, dass R = H ist.

6. $N^1$-3,4-Dimethoxybenzoyl-pyroglutaminsäure als neues technisches Produkt, Zwischenverbindung für die Synthese der Verbindung nach Anspruch 4.

7. Verbindung nach Anspruch 1, dadurch gekennzeichnet, dass R die 4-Methoxybenzoylgruppe bedeutet.

8. $N^1$-4-Methoxybenzoyl-pyroglutaminsäure als neues technisches Produkt, Zwischenverbindung für die Herstellung der Verbindung nach Anspruch 7.

9. Verbindung nach Anspruch 1, dadurch gekennzeichnet, dass R die 3,4-Dimethoxyphenylacetylgruppe bedeutet.

10. $N^1$-3,4-Dimethoxyphenylacetyl-pyroglutaminsäure als neues technisches Produkt, Zwischenverbindung für die Herstellung der Verbindung nach Anspruch 9.

11. Verbindung nach Anspruch 11, dadurch gekennzeichnet, dass R die Cyclopentylpropionylgruppe bedeutet.

12. $N^1$-Cyclopentylpropionyl-pyroglutaminsäure als neues technisches Produkt, Zwischenverbindung für die Herstellung der Verbindung nach Anspruch 11.

13. Verbindung nach Anspruch 1, dadurch gekennzeichnet, dass R die 2-Propylpentanoylgruppe bedeutet.

14. $N^1$-2-(Propyl)-pentanoyl-pyroglutaminsäure als neues technisches Produkt, Zwischenverbindung für die Herstellung der Verbindung nach Anspruch 13.

15. Verbindung nach Anspruch 1, dadurch gekennzeichnet, dass R für die 2-Ethylhexanoylgruppe steht.

16. $N^1$-2-(Ethyl)-hexanoyl-pyroglutaminsäure als neues technisches Produkt, Zwischenverbindung für die Herstellung der Verbindung nach Anspruch 15.

17. Verbindung nach Anspruch 1, dadurch gekennzeichnet, dass R die Hexanoylgruppe bedeutet.

18. $N^1$-Hexanoyl-pyroglutaminsäure als neues technisches Produkt, Zwischenverbindung für die Herstellung der Verbindung nach Anspruch 17.

19. Arzneimittel, bestehend aus oder enthaltend mindestens eine Verbindung nach Anspruch 1.

20. Verfahren zur Herstellung der Verbindungen nach Anspruch 1, dadurch gekennzeichnet, dass man die Natriumverbindung des Benzylesters der Pyroglutaminsäure mit einem Alkylierungs- oder Acylierungsmittel umsetzt, um die $N^1$-Alkyl- oder $N^1$-Acylverbindung des Benzylpyroglutamats zu erhalten, und dass man diese Verbindung nach der Hydrogenolyse mit Adamantanamin in Gegenwart eines Kupplungsmittels in einem inerten Lösungsmittel umsetzt.

21. Verfahren nach Anspruch 20, dadurch gekennzeichnet, dass man die $N^1$-Alkyl- oder $N^1$-Acylpyroglutaminsäure in ein reaktionsfähiges Derivat umwandelt, das anschliessend mit Adamantanamin in einem inerten Lösungsmittel, gegebenenfalls in Gegenwart eines Wasserstoffakzeptors umgesetzt wird.

22. Verfahren zur Herstellung der Verbindung nach Anspruch 5, dadurch gekennzeichnet, dass man ein reaktionsfähiges Derivat der $N^1$-Benzyloxy-carbonyl-pyroglutaminsäure herstellt und dieses auf Adamantanamin einwirken lässt und dass man anschliessend hydrogenolysiert, um die angestrebte Verbindung zu erhalten.

**Patentansprüche für den benannten Vertragsstaat: AT**

1. Verfahren zur Herstellung von N-(1-Adamantyl)-5-pyrrolidon-2-carboxamid-Verbindungen der allgemeinen Formel I

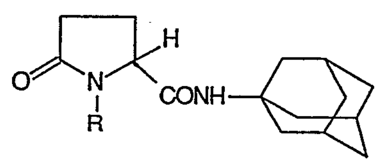

(I)

in der R für Wasserstoff oder eine lineare oder verzweigte Alkylgruppe mit 1 bis 7 Kohlenstoffatomen steht, die gegebenenfalls substituiert ist mit einer Alkoxyl-, Thioalkyl- oder Nitrilgruppe, oder für Acylgruppen vom Typ

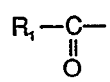 oder 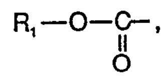,

wobei $R_1$ bedeutet:

- eine lineare oder verzweigte Alkylgruppe mit 1 bis 7 Kohlenstoffatomen,
- oder eine Cycloalkylgruppe mit 5 bis 7 Kohlenstoffatomen, die an die Carbonylgruppe über eine oder zwei Methylengruppen gebunden sein kann,
- oder einen aromatischen Kern, gegebenenfalls substituiert mit Substituenten, wie Nitrogruppe, $C_1$- bis $C_3$-Alkoxylgruppe, $C_1$- bis $C_3$-Alkylgruppe oder Halogenatom,
- eine Benzylgruppe, gegebenenfalls substituiert mit Substituenten, wie Halogenatom oder Nitro-, $C_1$- bis $C_3$-Alkoxyl- oder $C_1$- bis $C_3$-Alkylgruppen,

dadurch gekennzeichnet, dass man die Natriumverbindung des Benzylesters von Pyroglutaminsäure mit einem Alkylierungs- oder Acylierungsmittel umsetzt, um das $N^1$-Alkyl- oder $N^1$-Acylderivat des Benzylpyroglutamats zu erhalten und diese Verbindung nach der Hydrogenolyse mit Adamantanamin in Gegenwart eines Kupplungsmittels in einem inerten Lösungsmittel umsetzt.

2. Verfahren nach Anspruch 1, dadurch gekennzeichnet, dass man die $N^1$-Alkyl- oder $N^1$-Acylpyroglutaminsäure in ein reaktionsfähiges Derivat umwandelt, das anschliessend mit Adamantanamin in einem inerten Lösungsmittel, gegebenenfalls in Gegenwart eines Wasserstoffakzeptors umgesetzt wird.

3. Verbindung nach Anspruch 1 oder 2, dadurch gekennzeichnet, dass die Pyrrolidoncarboxamid-Gruppe die Konfiguration DL aufweist.

4. Verbindung nach Anspruch 1 oder 2, dadurch gekennzeichnet, dass die Pyrrolidoncarboxamid-Gruppe die Konfiguration L aufweist.